# EUROPEAN PATENT APPLICATION

(11) **EP 0 596 617 A1**
(43) Date of publication of application: **11.05.1994**
(21) Application number: 93308192.9
(22) Date of filing: 14.10.1993
(51) Int. Cl.: A61B 3/02, A61B 3/032

(54) **Vision testing**

(30) Priority: 02.11.1992 GB 9222908
(71) Applicant: EYELAB LIMITED, New Barnet, Herts EN5 1QG (GB)
(72) Inventor: Sheinman, John Stephen, Northampton NN1 2BP (GB); Field, Andrew, Woodbridge, Suffolk IP12 1BT (GB)
(74) Representative: Robinson, Nigel Alexander Julian

(57) **Abstract**

Apparatus for testing vision is described. This apparatus comprises a computer 2 having a display 4 for displaying a sequence of vision testing patterns (Figures 5 to 9) to effect a sequence of differing visual tests. A test subject inputs responses via a keyboard 8. The responses are analysed to give an indication 54 of defective vision. A screen filter 10 and a pair of filtering spectacles 14 cooperate to isolate the test subject's eyes by allowing differing portions of the vision testing patterns to be observed only by one of the test subject's eyes. The screen filter 10 and the pair of spectacles 14 are formed of polarising filters.

## Description

This invention relates to vision testing.

The testing of a patient's vision to detect and diagnose any abnormalities or defects is a well established practice for which many differing types of vision testing apparatus are known. Typically, such vision testing apparatus is bulky, requires expert knowledge to use effectively and, due to its specialist nature, is expensive. A practising Optometrist (Ophthalmic Optician) will generally have a dedicated room in which their vision testing apparatus is permanently installed for use in testing patients who visit the Optometrist's premises.

With the increasingly widespread use of computers within all walks of life, concern has been raised over the demands that the use of such equipment places upon the visual performance of computer operators. The extent of this concern is such that it is considered desirable (and may soon be legally required) that computer operators should undergo regular visual testing to assess their initial and continuing suitability for using computer equipment. The costs in terms of lost working time, inconvenience and professional charges of having such testing performed by an Optometrist would be considerable. In the case of companies employing hundreds or thousands of computer operators, such a testing programme could be hugely expensive.

Viewed from a first aspect this invention provides apparatus for testing vision, said apparatus comprising:
a computer having a display for displaying a sequence of vision testing patterns to effect a sequence of differing visual tests, a user input device for inputting test subject responses to said vision testing patterns and means for detecting whether said test subject responses are indicative of vision unsuitable for computer display operation;
means for holding a first filter in front of a first eye of a test subject and a second filter in front of a second eye of said test subject, at least one of said vision testing patterns including a first area obscured from said first eye by said first filter and a second area obscured from said second eye by said second filter; and
a screen filter disposed in front of said display, said screen filter having a first complementary filter area for passing light that is passed by said first filter and obscured by said second filter, a second complementary filter area for passing light that is passed by said second filter and obscured by said first filter and an unfiltered area.

The invention recognises and exploits that the computer equipment which is causing the above mentioned concern can itself be used to perform a screening test (a measure of visual performance) without the need for the bulky and expensive equipment usually used by Optometrists or indeed the initial involvement of an Optometrist at all. The cost savings provided by the invention make mass screening programmes to test subject's suitability for computer display operation a more attractive proposition.

In addition to the above, the use of the computer equipment itself to perform the tests has the advantage that the tests are performed in an environment that closely matches the day to day use of the computer equipment that is made. In particular, if a computer operators own computer is used to perform the testing, then conditions such as ambient lighting, seating position and posture, etc. can be made identical to those found in practice.

It will be appreciated that in order to achieve an acceptably accurate screening test, a variety of differing vision testing patterns will be displayed, each intended to test an aspect such: as acuity; more than 50% monocular visual impairment; eye coordination; visual field; colour vision; ability to accommodate; eye tracking; latent hyperopia or the like. The display and data storage facilities already present within the computer being used, allow such a variety of differing vision testing patterns to be generated without difficulty. Furthermore, the user input devices, such as a keyboard that are already associated with the computer can be used to receive the test subject's responses to the tests and the data processing capabilities of the computer used to analyse these responses.

In order to use some vision testing patterns it is necessary to isolate the performance of each of a test subject's eyes whilst allowing all tests to be conducted binocularly so as to more fully simulate normal viewing conditions. To this end, there is provided means for holding a first filter in front of a first eye of a test subject and a second filter in front of a second eye of said test subject, with at least one of said vision testing patterns including a first area obscured from said first eye by said first filter and a second area obscured from said second eye by said second filter.

It will be appreciated that one possibility might be to employ a direct filtering of different coloured regions displayed on the computer display so as to isolate each of the test subject's eyes, e.g. some characters could be displayed in green and other characters displayed in red, with red and green coloured filters held over the test subject's eyes. However, the performance of such direct filtering would be subject to degradation due to the colours that were produced by a particular computer display not being closely enough matched to the filters in front of the test subject's eyes.

Accordingly, embodiments of the invention includes a screen filter disposed in front of said display, said screen filter having a first complementary filter area for passing light that is passed by said first filter and obscured by said second filter, a second complementary filter area for passing light that is passed by said second filter and obscured by said first filter and an unfiltered area.

Operational instructions or tests requiring both eyes can be displayed through this unfiltered area. The use of such a screen filter allows accurate characteristics to be imposed upon the light from different parts of the vision testing patterns, which in turn improves the filtering and degree of isolation that can be achieved.

It will be appreciated that the use of a screen filter to some extent reduces the versatility of the display for displaying differing vision testing patterns. However, in preferred embodiments of the invention a border separating said first complementary filter area from said second complementary filter area includes a substantially horizontal portion and a substantially vertical portion. Such screen filters allow sufficient versatility to enable an effective set of screening tests to be performed.

A simple and effective implementation of the means for holding is one in which said means for holding comprises an oversized pair of spectacles. The oversized nature of these spectacles allow a user's normal spectacles, if any, to be worn beneath the oversized spectacles.

In order to allow a test to be made of the test subject's ability to accommodate, a pair of oversized spectacles having a non-zero correction are also provided.

As mentioned above, the filtering that is performed to isolate a test subject's eyes can be based upon the selective filtering of different colours. However, in preferred embodiments of the invention said first filter, said second filter, said first complementary filter area and said second complementary filter area are polarising filters. The use of polarising filters allows effective isolation to be achieved without introducing an unnatural variation in the colour of the vision testing patterns being observed.

The analysis of a test subject's responses to the differing vision testing patterns is one that should ideally be performed by an experienced Optometrist since the accurate identification of a particular problem may depend on many complicated factors such as the age of the test subject, family background or other accompanying medical conditions. However, if the testing performed by the invention is regarded as screening to identify test subjects who require a full examination by such an experienced Optometrist, then advantageously simple embodiments of the invention may be such that said means for detecting compares said test subject responses with predetermined ranges of normal values and indicates a failure of a screening test if one or more of said test subject responses lie outside said predetermined ranges. It will be appreciated that the invention may be considered to also provide a new method of testing vision and a kit of parts for use with a computer to adapt it into a vision testing apparatus.

Viewed from a second aspect this invention provides a vision testing kit comprising:
means for holding a first filter in front of a first eye of a test subject and a second filter in front of a second eye of said test subject; and
a screen filter for disposing in front of a computer display, said screen filter having a first complementary filter area for passing light that is passed by said first filter and obscured by said second filter, a second complementary filter area for passing light that is passed by said second filter and obscured by said first filter and an unfiltered area.

Viewed from a third aspect this invention provides a screen filter for disposing in front of a computer display, said screen filter having a first filter area having a first light transmission characteristic, a second filter area having a second light transmission characteristic complementary to said first light transmission characteristic and an unfiltered area.

Viewed from a fourth aspect this invention provides a oversized pair of spectacles having a first filter lens with a first light transmission characteristic and a second filter lens with a second light transmission characteristic complementary to said first light transmission characteristic, said first filter lens and said second filter lens both having non-zero correction.

An embodiment of the invention will now be described, by way of example only, with reference to the accompanying drawings in which:
Figure 1 illustrates a vision testing apparatus according to one embodiment of the invention;
Figure 2 illustrates a screen filter;
Figure 3 illustrates a pair of spectacles with filter lenses;
Figure 4 illustrates a display screen for assisting alignment of the screen filter;
Figures 5 to 10 illustrate various vision testing patterns; and
Figure 11 illustrates an analysis of test subject responses.

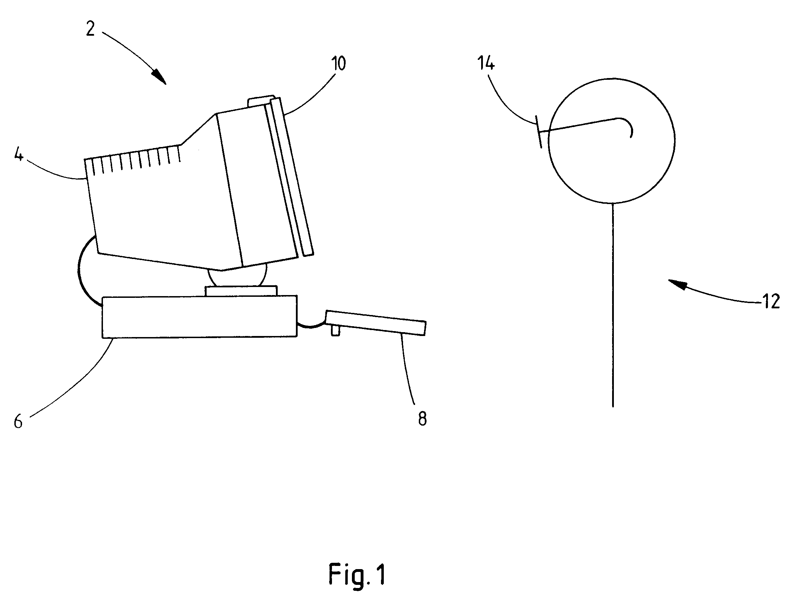

Figure 1 illustrates a vision testing apparatus comprising a computer 2 having a display 4, a system unit 6 and a keyboard 8. A screen filter 10 is clipped in position in front of the display 4. A test subject 12 to be screened wears a pair of spectacles 14 having filter lenses.

In use, the test subject enters their personal details, such as name, sex and age, via the keyboard 8. This information is stored by the computer 2 along with the results of the tests to be performed. The computer 2 then displays a sequence of vision testing patterns upon the display 4. Data defining these vision testing patterns is contained in the program controlling the system unit 6. The test subject 12 observes the displayed vision testing patterns through the spectacles 14 and the screen filter 10. Following instructions that appear on the display, the test subject 12 enters their responses to the vision testing patterns via the keyboard 8. The test subject responses are stored by the system unit 6. At the end of the sequence of vision testing patterns, the system unit 6 references the stored test subject responses and compares these with ranges of normal values. If the test subject responses are outside these ranges then a message is displayed advising the test subject to see an Optometrist and a note of the failure of the screening test is made against the test subject's name. Alternatively, if the test subject passes the screening test a message is displayed indicating a pass and a note of the pass made against the test subject's name.

A single computer can be loaded with the data and programs necessary to run the screening test for a number of test subjects, or alternatively the screening test could be loaded onto each individual computer user's computer.

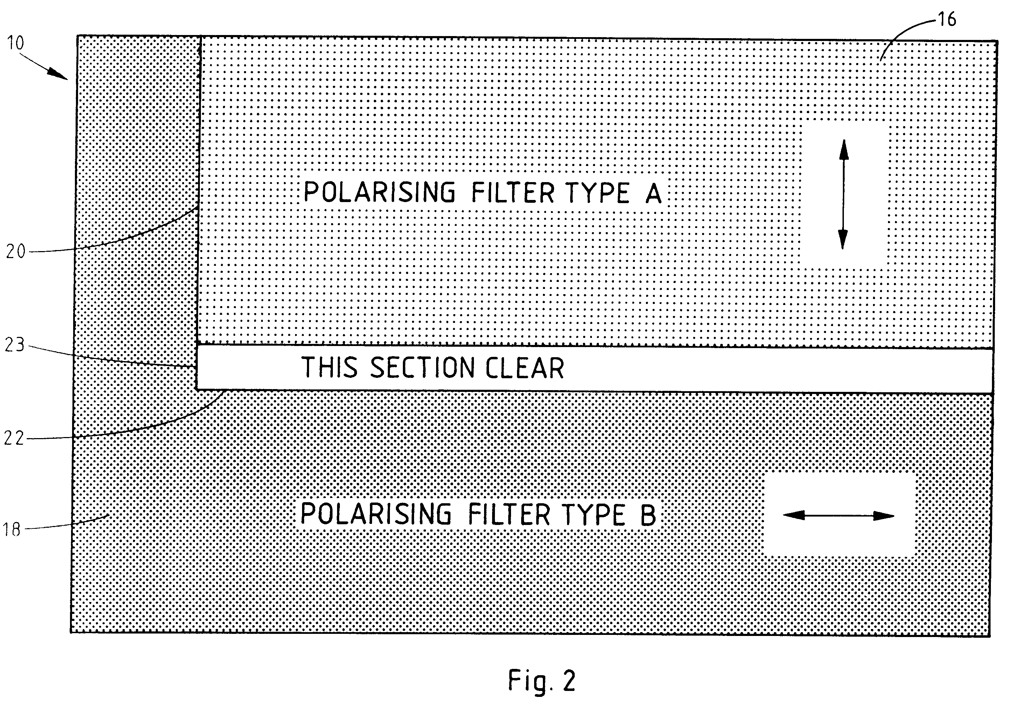

Figure 2 illustrates the screen filter 10 in more detail. The screen filter 10 has a first complementary filter area 16 and a second complementary filter area 18. The first complementary filter area 16 comprises a sheet of polarising filter with the transmission direction running in a first direction (in this particular example vertically). The second complementary filter 18 comprises a sheet of polarising filter with the transmission direction running in a second direction substantially perpendicular to the first direction (in this particular example horizontally). The border between the first complementary filter and the second complementary filter includes a vertical portion 20 and a horizontal portion 22.

A clear (unfiltered) area 23 is provided between the first complementary filter 16 and the second complementary filter 18. The clear area can be used to display instructions to the test subject or for tests requiring the use of both eyes. The clear area 23 is vertically centred in the screen filter 10 and occupies approximately 5% of the screen height.

The first complementary filter 16 extends from the top of the screen to the clear area 23 and is approximately 85% of the total screen width. The second complimentary filter 18 extends from the top left of the screen occupying approximately 15% of the screen width with the bottom section extending over the full width of the screen.

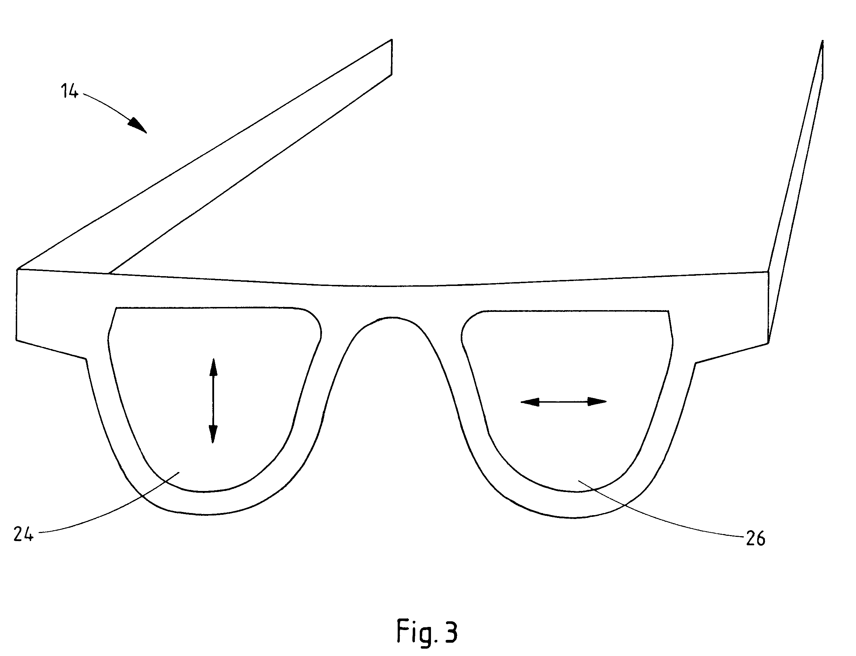

Figure 3 illustrates a pair of oversized spectacles 14 that are worn by the test subject (possibly over their own spectacles). The right lens of the spectacles 14 is a first filter 24 comprising a polarising filter with a vertical transmission direction. The left lens of the spectacles 14 is a second filter 26 comprising a polarising filter with a horizontal transmission direction. The combined action of the spectacles 14 and the screen filter 10 allows areas of the test pattern beneath the first complementary filter areas 16 to be viewed through the right eye and areas below the second complementary filter area 18 to be viewed through the left eye.

A second pair of oversized spectacles 14 is provided that is the same as the above except that a non-zero correction of -2.25 dioptres is provided for each eye. This allows the test subject's ability to accommodate to be tested.

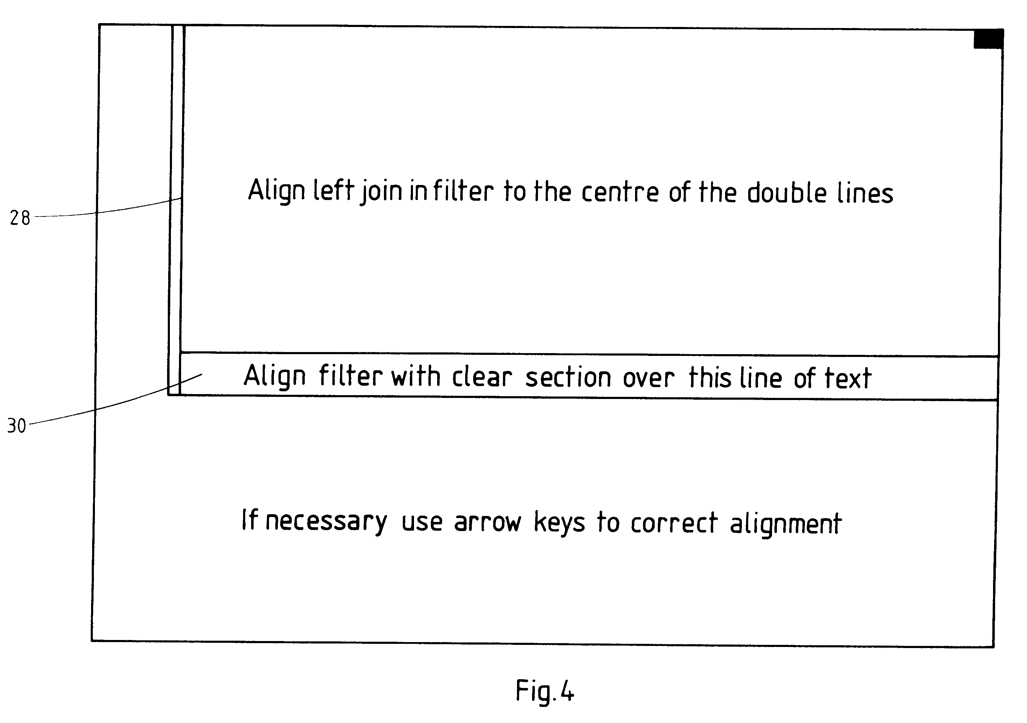

Figure 4 illustrates a screen that is displayed upon the display 4 to enable the screen filter 10 to be accurately positioned relative to the display 4. Following the instructions on the screen, the user adjusts the position of the screen filter 10 such that the vertical portion 20 of the border overlays the vertical guideline 28 and the clear section 23 overlays the horizontal guide 30. The positioning can be fine tune using the arrow cursor keys on the keyboard 8 to define an offset to be applied to the vision testing patterns to be displayed. It is important that the screen filter 10 should be accurately positioned relative to the display 4 so that the vision testing patterns subsequently displayed will have appropriate portions obscured to respective eyes by the combined action of the screen filter 10 and the spectacles 14.

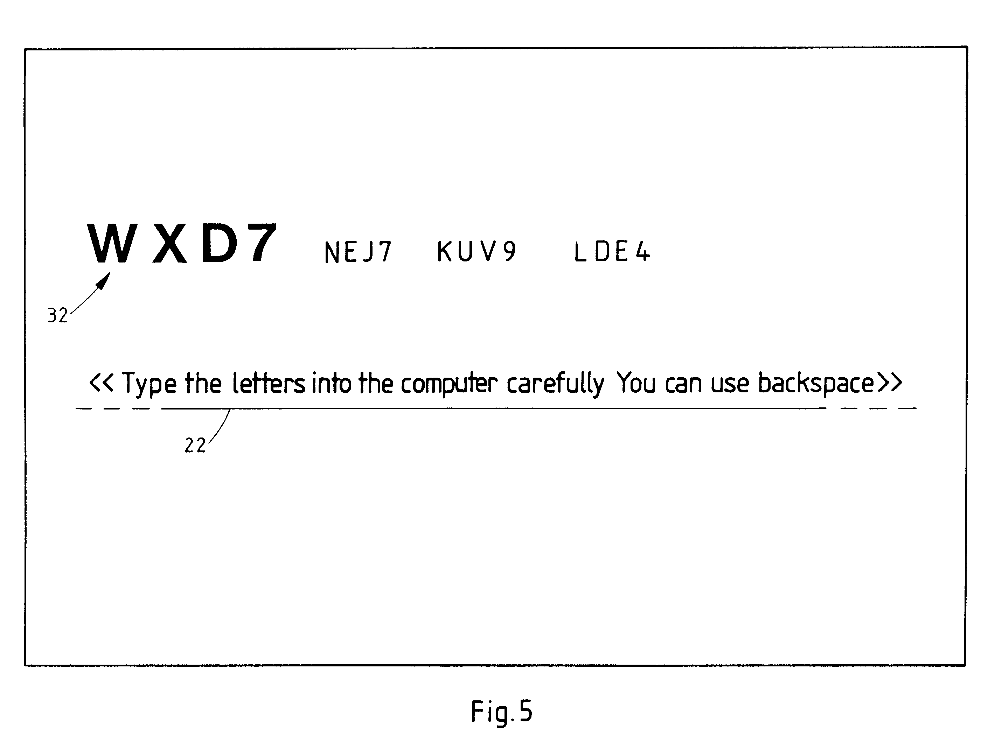

Figure 5 illustrates a vision testing pattern for testing visual acuity. A sequence of ophthalmic Snellen characters 32 are displayed upon a region above the horizontal portion 22 of the border where they may be viewed by the test subject's right eye. The test subject is asked to enter, via the keyboard 8, the sequence of characters observed. The computer 2 compares the test subject responses with the sequence of characters it is displaying to determine if the characters are being correctly identified. Eye dominance and long-standing monocular visual impairment by greater than 50% is revealed. Successively smaller sequences of characters 32 are displayed to assess the limit of the test subject's visual acuity with the right eye. The process can then be repeated with characters displayed below the horizontal portion 22 of the border to assess the visual acuity of the test subject's left eye.

The use of the computer means that the string of characters 32 can be readily made to have a randomised order so guarding against the possibility of a test subject memorising the sequence.

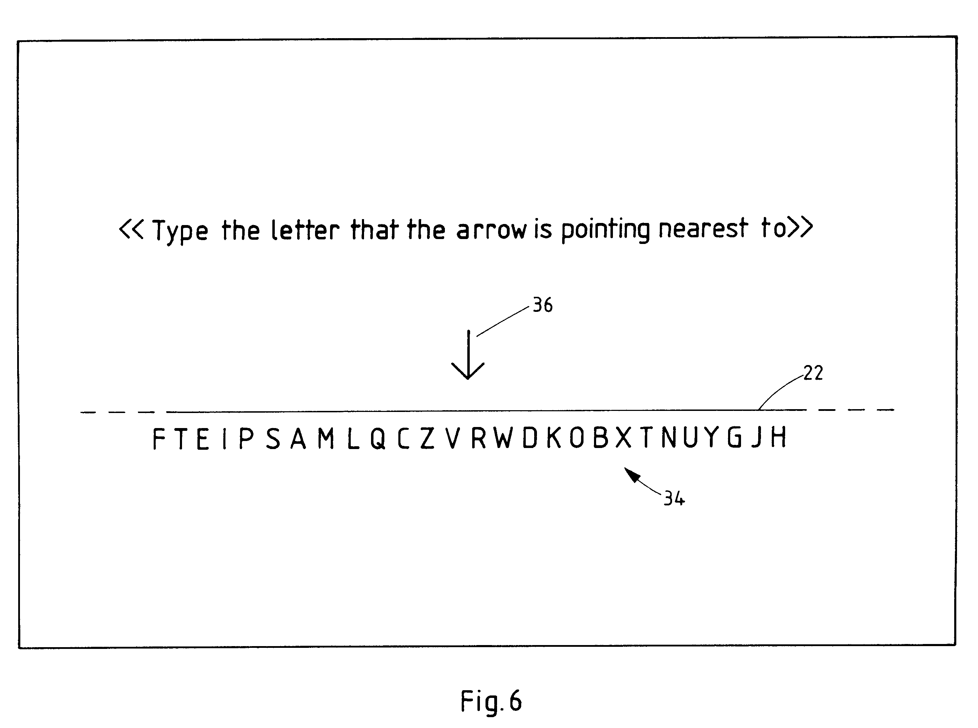

Figure 6 illustrates a vision testing pattern for assessing the horizontal extra-ocular muscle balance (horizontal coordination) of the eyes. A sequence of letters 34 is displayed above the horizontal portion 22 of the border where they may be observed only by the right eye. A marker 36 is displayed below the horizontal portion 22 of the border where it may be observed only by the left eye. The test subject inputs via the keyboard 8 the letter within the sequence of letters 34 that they observe to most closely align with the marker 36. A deviation from the actual alignment in the vision testing pattern would be indicative of inadequate horizontal coordination of the eyes.

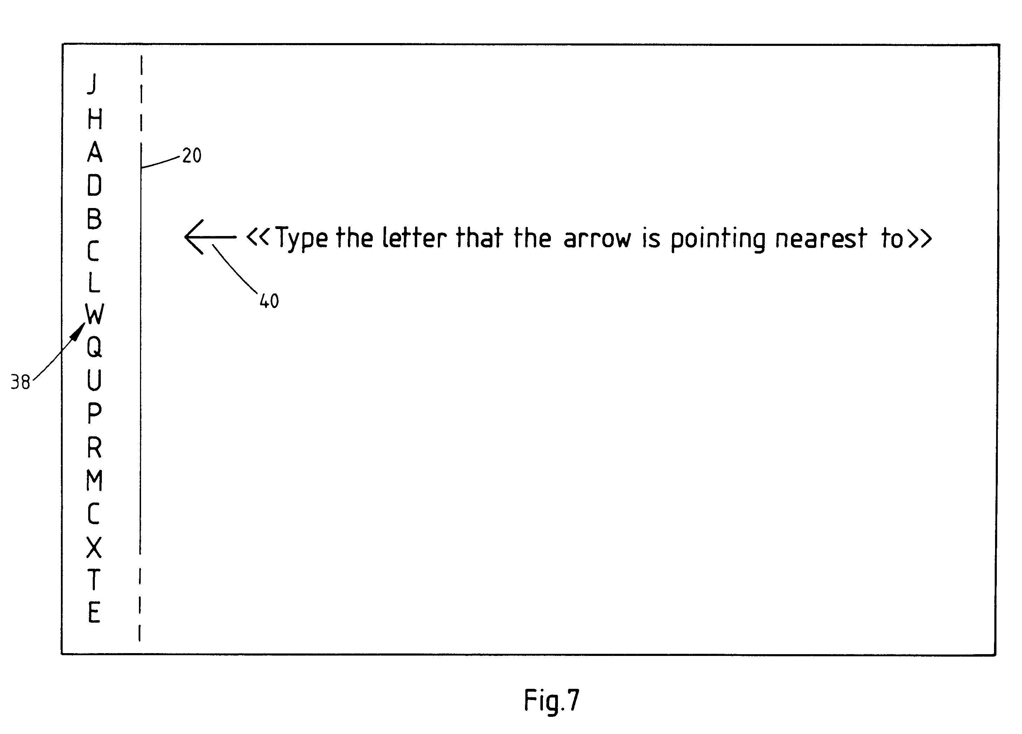

Figure 7 illustrates a test pattern similar to that of Figure 6, but in this case testing vertical coordination. A column of characters 38 are displayed on the left side of the vertical portion 20 of the border where they may only be observed by the test subject's left eye. A marker 40 is displayed on the right side of the vertical portion 20 of the border where it may only be observed by the test subject's right eye. The test subject types the letter within the column of characters 38 that they observe to align with the marker 40. Any deviation from the letter which in fact aligns with the marker 40 indicates an inadequate vertical coordination of the test subject's eye. These muscle balance tests are subject to the natural binocular lock of the screen frame and workplace environment refining these co-ordination tests into fixation disparity evaluations.

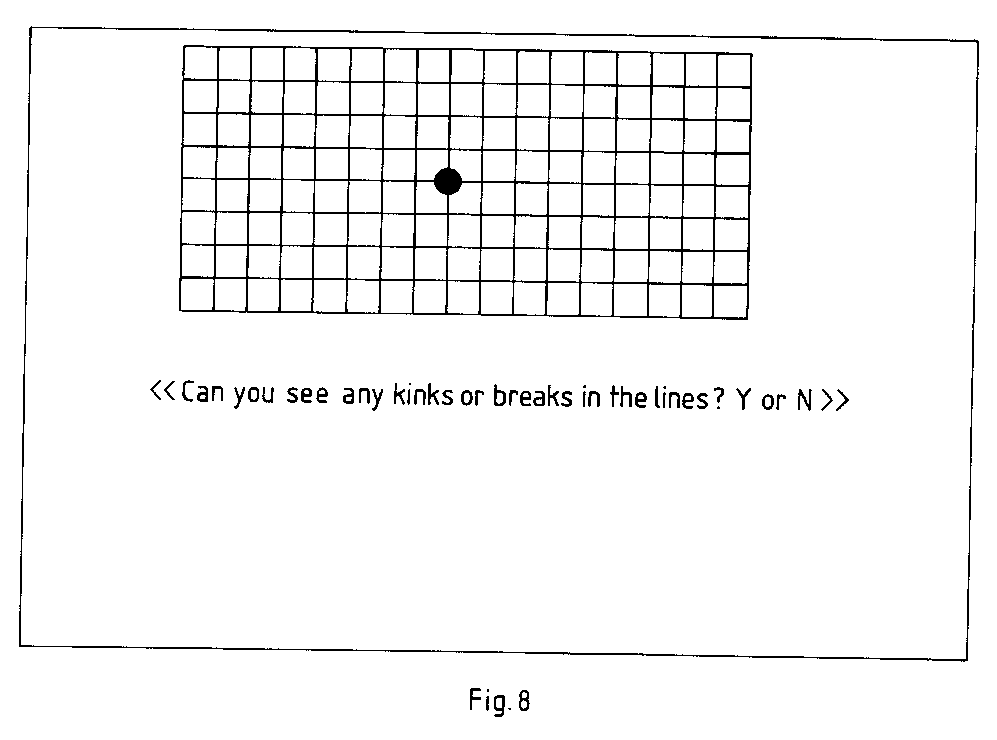

A vision testing pattern corresponding to an Amsler chart is then displayed as illustrated in Figure 8. This Amsler chart can be displayed first behind the first complementary filter area 16 to test the test subject's right eye and then behind the second complementary filter area 18 to test the test subject's left eye. In each instance the test subject is asked to input via the keyboard 8 whether the horizontal and vertical lines appear straight and unbroken.

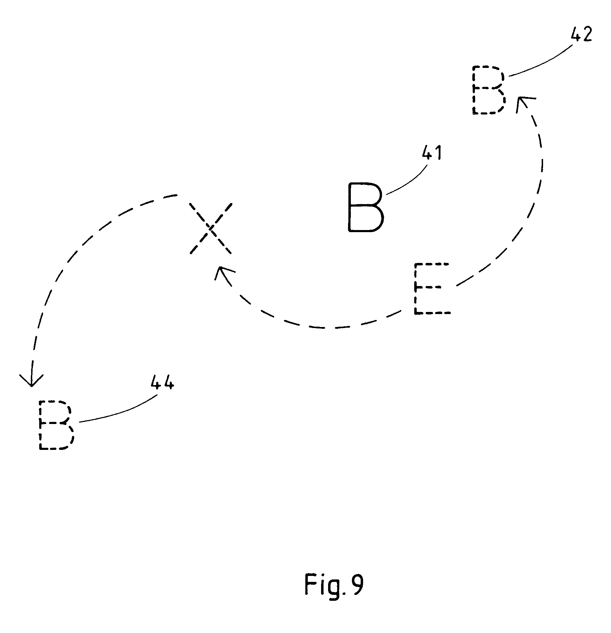

Figure 9 illustrates an eye tracking test. A central character 41 is displayed in a central portion of one of the areas behind the first complementary filter area 16 (to test the right eye) and the second complementary filter area 18 (to test the left eye). Various characters are then briefly display in the area surrounding the central character 41. If the characters match (e.g. characters 41 and 42, and 41 and 44), then the test subject presses a key on the keyboard 8 to indicate this. By measuring the success with which the test subject is able to notice the matching characters, an indication of an eye's tracking ability can be made. This test is first performed for one eye and then for the other eye.

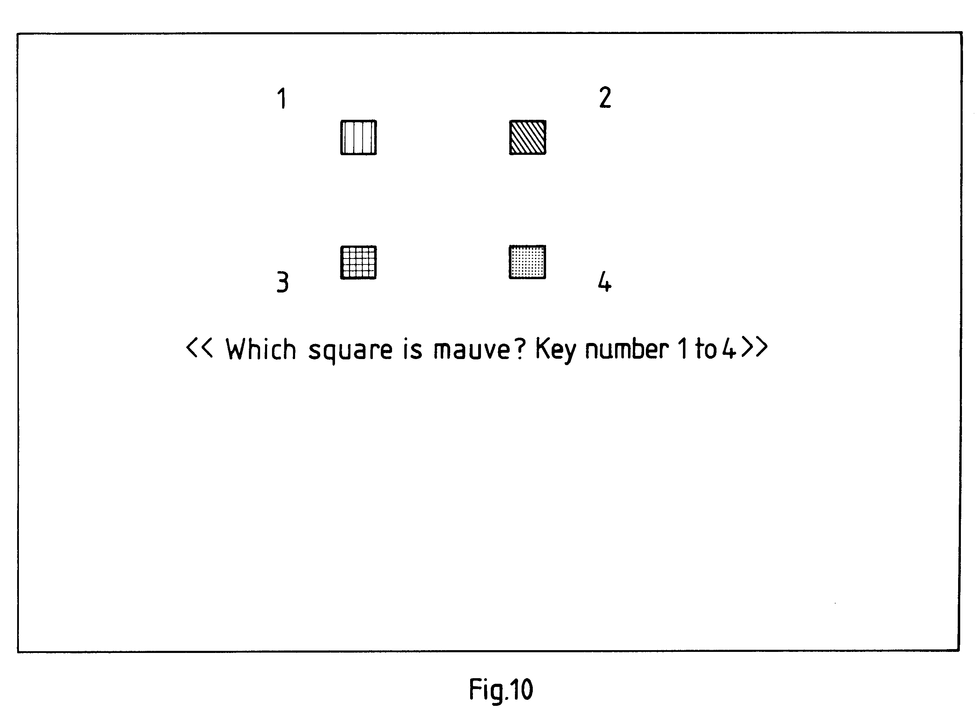

If a colour display 4 is available then the test pattern illustrated in Figure 10 can be used to assess the test subject's colour vision. This test may in some circumstances not be considered relevant for the screen test.

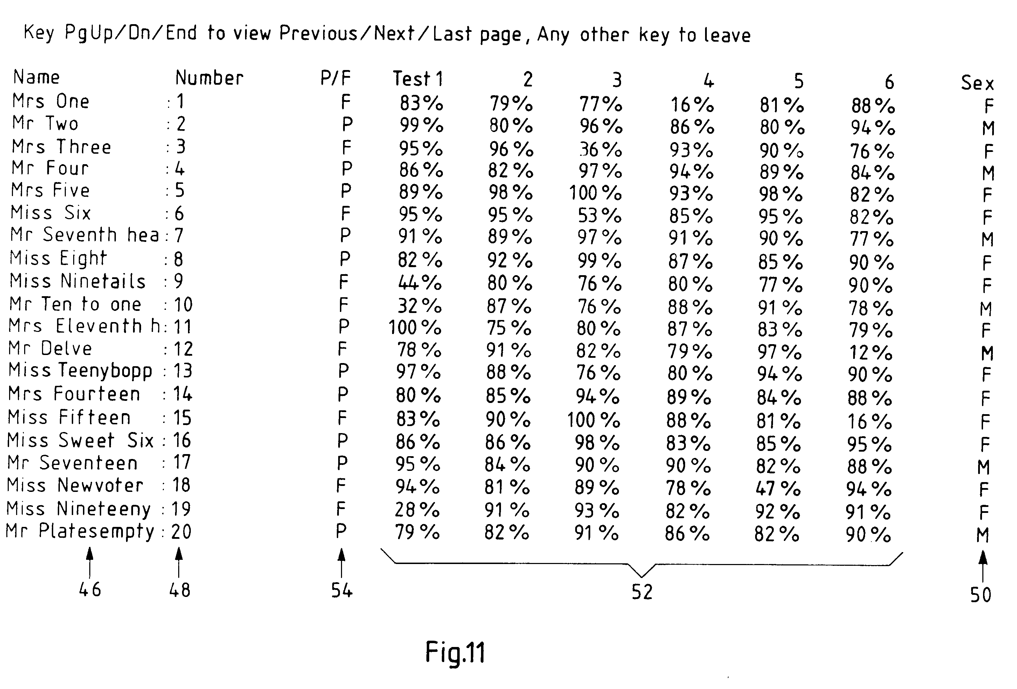

Figure 11 illustrates a tabulated set of screening test results that is determined by the computer 2. Test subject details are given in columns 46, 48 and 50. The individual test results are given in the group of columns 52 and a pass/fail indication is given in column 54. The pass/fail indication as well as an explanation of their performance relative to each characteristic being measured would be given to each test subject at the end of their screening test. Those test subjects that have failed the screening test would then go to an Optometrist for a more detailed analysis. It will be appreciated that in at least preferred embodiments of the invention a test subject's visual perfqrmance is measured, analyzed and recorded for future reference.

## Claims

1. Apparatus for testing vision, said apparatus comprising:
a computer (2) having a display (4) for displaying a sequence of vision testing patterns to effect a sequence of differing visual tests, a user input device (8) for inputting test subject responses to said vision testing patterns and means for detecting whether said test subject responses are indicative of vision unsuitable for computer display operation;
means (14) for holding a first filter (24) in front of a first eye of a test subject and a second filter (26) in front of a second eye of said test subject (12), at least one of said vision testing patterns including a first area obscured from said first eye by said first filter and a second area obscured from said second eye by said second filter; and
a screen filter (10) disposed in front of said display, said screen filter having a first complementary filter area (16) for passing light that is passed by said first filter and obscured by said second filter, a second complementary filter area (18) for passing light that is passed by said second filter and obscured by said first filter and an unfiltered area (23).

2. Apparatus as claimed in claim 1, wherein said means for holding comprises an oversized pair of spectacles.

3. Apparatus as claimed in claim 2, wherein said oversized spectacles have non-zero correction.

4. Apparatus as claimed in any one of claims 1, 2 and 3 wherein a border separating said first complementary filter area from said second complementary filter area includes a substantially horizontal portion (22) and a substantially vertical portion (20).

5. Apparatus as claimed in any one of the preceding claims wherein said first filter, said second filter, said first complementary filter area and said second complementary filter area are polarising filters.

6. Apparatus as claimed in any one of the preceding claims, wherein said means for detecting compares said test subject responses with predetermined ranges of normal values and indicates a failure of a screening test if one or more of said test subject responses lie outside said predetermined ranges.

7. A vision testing kit comprising:
means (14) for holding a first filter (24) in front of a first eye of a test subject and a second filter (26) in front of a second eye of said test subject; and
a screen filter (10) for disposing in front of a computer display (4), said screen filter having a first complementary filter area (16) for passing light that is passed by said first filter and obscured by said second filter, a second complementary filter area (18) for passing light that is passed by said second filter and obscured by said first filter and an unfiltered area (23).

8. A vision testing kit as claimed in claim 7, comprising a data storage medium bearing a computer program from controlling a computer (2) to display a sequence of vision testing patterns upon a computer display to effect a sequence of differing visual tests, receive test subject responses via a user input device (8) to said vision testing patterns, and detect whether said test subject responses are indicative of vision unsuitable for computer display operation.

9. A screen filter (10) for disposing in front of a computer display (4), said screen filter having a first filter area (16) having a first light transmission characteristic, a second filter area (18) having a second light transmission characteristic complementary to said first light transmission characteristic and an unfiltered area (23).

10. An oversized pair of spectacles having a first filter lens (24) with a first light transmission characteristic and a second filter lens (26) with a second light transmission characteristic complementary to said first light transmission characteristic, said first filter lens and said second filter lens both having non-zero correction.
